Europäisches Patentamt

European Patent Office

Office européen des brevets

(11) Publication number: **0 348 091 B1**

# EUROPEAN PATENT SPECIFICATION

(45) Date of publication of patent specification: **12.01.94** (51) Int. Cl.5: **C09K 3/30**

(21) Application number: **89305990.7**

(22) Date of filing: **13.06.89**

(54) Aerosol composition.

(30) Priority: **20.06.88 JP 150194/88**
**11.10.88 JP 255401/88**

(43) Date of publication of application:
**27.12.89 Bulletin 89/52**

(45) Publication of the grant of the patent:
**12.01.94 Bulletin 94/02**

(84) Designated Contracting States:
**DE ES FR GB IT NL**

(56) References cited:
**EP-A- 0 251 462**
**GB-A- 2 029 441**
**US-A- 3 494 871**
**US-A- 4 328 319**

**SEIFEN-OLE-FETTE-WACHSE, vol. 98, no. 15,
20th July 1972, pp. 489-491; W. LANZENDORF:
"Aerosole-technische Hinweise zu
Puderaerosolen und Aerosol-Treibmitteln"**

(73) Proprietor: **SHISEIDO COMPANY LIMITED**
**5-5 Ginza 7-chome**
**Chuo-ku Tokyo 104(JP)**

(72) Inventor: **Moro, Osamu c/o Shiseido Laborato-
ries**

**1050, Nippa-cho**
**Kohoku-ku**
**Yokohama-shi Kanagawa(JP)**
Inventor: **Tamaki, Shuya c/o Shiseido Labora-
tories**
**1050, Nippa-cho**
**Kohoku-ku**
**Yokohama-shi Kanagawa(JP)**
Inventor: **Nakane, Toshihiko c/o Shiseido Lab-
oratories**
**1050, Nippa-cho**
**Kohoku-ku**
**Yokohama-shi Kanagawa(JP)**
Inventor: **Iwai, Tsunehiko c/o Shiseido Lab-
oratories**
**1050, Nippa-cho**
**Kohoku-ku**
**Yokohama-shi Kanagawa(JP)**

(74) Representative: **Adams, William Gordon et al**
**RAWORTH, MOSS & COOK**
**36 Sydenham Road**
**Croydon Surrey CR0 2EF (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 348 091 B1

EP 0 348 091 B1

**Description**

The present invention relates to an aerosol composition having a very good dispersibility and an excellent useability due to a composite powder formulated therein. The aerosol composition of the present invention can be utilized in such fields as drugs, quasi-drugs, cosmetics, sterilizers, and lubricants.

Usually, a powder spray comprising a powder dispersed in a liquefied gas repellant is used as a dry shampoo, food powder, sterilizer, lubricator, and dry type antisweat agent. The dry type antisweat agent presently used is widely sold on the market.

A chlorofluoro carbon gas is widely used as the propellant for these powder sprays, due to its ease of handling.

Currently, because of the influence of this gas on the ozone layer, there is movement to restrict the use of chlorofluoro carbon gas, and the present trend is toward a reduction of the amount of chlorofluoro carbon gas used or finding a substitute therefor.

The powder spray gas generally contains, as the lubricator, talc, mica, silica, and spherical nylon, as a sweat preventive and deodorant, and powder of aluminum chloride, aluminum hydroxychloride, zinc oxide, and hydroxyapatite formulated therein.

If, however, the use of chlorofluoro carbon (i.e., "CFC") gas as the propellant is restricted, the dispersibility of the powder is poor when these powders are formulated, and even when used after strong shaking, the powder may be sedimented during spraying, and thus it is not possible to ensure spraying for a relatively long period, of a uniform composition.

The prior art contains many references to powder aerosol compositions, for example:

(i) Seifen-Ole-Fette-Wachse, Vol. 98, No.15, 20th July 1972, pages 489-491, by W. Lanzendorf discloses a powder aerosol composition containing a mixed powder.

(ii) GB Patent No. 2,029,441 discloses a powder aerosol composition comprising a suspension of powder, an alcohol, water, a propellant.

(iii) US Patent No. 3,494,871 discloses a package chemiluminescent aerosol containing a normally gaseous propellant, a luminescent agent and a finely divided solid particle diluent.

(iv) EP No. 0,251,462 discloses a polymer-propellant composition containing a polymer and a propellant.

(v) US Patent No. 4,328,319 discloses a propellant composition including a film forming synthetic polymer and a propellant.

However, neither the use of the composite powder specified in the present invention nor the desirable results obtained therefrom are taught in these citations.

Accordingly, the present invention seeks to obviate the above-mentioned problems and to provide an aerosol composition having an excellent dispersibility and useability even if chlorofluoro carbon gas is not used or the amount of the chlorofluoro carbon gas is restricted.

In accordance with the present invention, there is provided an aerosol composition comprising at least one propellant selected from the group consisting of propane, isobutane, n-butane, and a liquefied petroleum gas (abbreviated hereinafter as "LPG") which is a mixture thereof, a chlorinated hydrocarbon and dimethyl ether, and a composite powder, the composite powder comprising a core powder having an average particle size of 0.1 to 100 $\mu$m consisting essentially of a polymer and a sheath powder having an average particle size of 1/5 or less of that of the core powder attached to the surface of the core powder.

In accordance with the present invention, there is also provided an aerosol composition comprising at least one propellant selected from the group consisting of propane, n-butane, isobutane and a liquefied petroleum gas, at least one solvent selected from the group consisting of n-pentane, isopentane, and n-hexane, and a composite powder comprising a core powder having an average particle size of 0.1 to 100 $\mu$m consisting essentially of a polymer and a sheath powder having an average particle size of 1/5 or less of that of the core powder attached to the surface of the core powder.

The composite powder usable in the present invention comprises one or more coating powder which becomes a sheath (hereinafter abbreviated as sheath powder) attached as a composite around the powder which becomes the core (hereinafter abbreviated as the core powder). The sheath powder can be selected from among inorganic powders and organic powders as desired.

As the core powder of the composite powder usable in the present invention, any desired organic powder with an average particle size of 0.1 to 100 $\mu$m can be used, and the powder used for the core can be in the form of a spherical, plate, granular, or needle, but in view of the feeling on the skin during use of the aerosol compositions, the spheroid, granular or plate form is preferred.

Typical examples of the core powder of the composite powder are polyamides such as nylon 6, nylon 12, nylon 46, and nylon 66; polyolefins such as polyethylene and polypropylene; polystyrene; polyesters such as polyethylenenterephthalate and polycarbonate; acrylic resins such as polymethyl methacrylate and

2

EP 0 348 091 B1

butyl acrylate-MMA copolymer; epoxy resins such as cured bisphenol A-epichlorohydrin copolymer; fluorine type resins such as polytetrafluoroethylene; silicone type resins such as methylpolysioxane and crosslinked dimethylpolysiloxane; phenolic resins such as benzoguanamine resin; vinyl resins such as polyvinyl chloride and polyvinyl methyl ether; polyurethane; cellulose; chitin; chitosan; fibroin; keratin; natural rubber; and so on.

The sheath powder, which is attached to the core powder to form the comosite powder may have an average particle size of 1/5 or less, preferably 1/10 or less, of that of the core powder. If the average particle size of the sheath powder is larger than 1/5 of the average particle size of the core powder, the elimination stability of the sheath powder will be extremely poor.

Typical examples of the sheath powder usable in the present invention include, as inorganic powders in general, anhydrous silicic acid, magnesium silicate, talc, kaolin, bentonite, aerosil, mica, mica titanium, bismuth oxychloride, zirconium oxide, magnesium oxide, zinc oxide, titanium oxide, light calcium carbonate, heavy calcium carbonate, light magnesium carbonate, heavy magnesium carbonate, yellow iron oxide, red iron oxide, black iron oxide, ultramarine, carbon black, caramine hydroxyapatite, magnesium aluminometasilicate, magnesium aluminosilicate, aluminum hydroxychloride, aluminum chloride, aluminum sulfate, aluminum citrate, aluminum acetate, basic aluminum chloride, aluminum phenolsulfonate, aluminum $\beta$-naphtholdisulfonate, lead acetate, sodium perborate, aluminum zirconium octachlorohydrate, aluminum zirconium pentachlorohydrate, aluminum zirconium tetrachlorohydrate, aluminum zirconium trichlorohydrate, and zirconium chlorohydrate; as organic pigments, pearl pigments such as mica coated with titanium oxide, bismuth oxychloride coated with titanium oxide, talc coated with titanium oxide, fish scale, mica coated with colored titanium oxide; metal powder pigments such as aluminum powder and copper powder; organic pigments such as Red color No. 201, Red color No. 202, Red color No. 204, Red color No. 205, Red color No. 220, Red color No. 226, Red color No. 228, Red color No. 405, Orange color No. 203, Orange color No. 204, Yellow color No. 205, Yellow color No. 401, and Blue color No. 404; zirconium, barium or aluminum lake such as Red color No. 3, Red color No. 104, Red color No. 106, Red color No. 227, Red color No. 230, Red color No. 401, Red color No. 505, Orange color No. 205, Yellow color No. 4, Yellow color No. 5, Yellow color No. 202, Yellow color No. 203, Green color No. 3 and Blue color No. 1; as organic powders, polyamides such as nylon 6, nylon 12, and nylon 66; polyolefins such as polyethylene and polypropylene; polystyrene; polyesters such as polyethyleneterephthalate and polycarbonate; acrylic resins such as polymethyl methacrylate and butyl acrylate-MMA copolymer; epoxy resins such as cured bisphenol A-epichlorohydrin copolymer; fluorine type resins such as polytetrafluoroethylene; silicone type resins such as methylpolysioxane and crosslinked dimethylpolysiloxane; phenolic resins such as benzoguanamine resin; vinyl resins such as polyvinyl chloride and polyvinyl methyl ether; polyurethane; cellulose; chitin; chitosan; fibroin; keratin; natural rubber; metalic soaps such as aluminum stearate, calcium stearate, magnesium stearate, zinc stearate, aluminum myristate, calcium, myristate, magnesium myristate, zinc myristate, aluminum palmitate, calcium palmitate, magnesium palmitate, zinc palmitate, zinc laurate, zinc undecylenate and calcium acetate; chlorophyll; tannin powder, and flavonoid.

As the method of preparing the composite powder to be used in the present invention, for example, the composite powder can be prepared by mixing the core powder and the sheath powder by the dry process or the wet process. As the mixing device, there may be employed various mixing devices or pulverization devices such as a ball mill, pot mill, automated mortar, grinder, attritor, sand mill, ongmill, hybridizer, cosmomizer, mechanofusion system, and hybridization system.

The mixing ratio of the core powder to the sheath powder may be, in terms of weight ratio, 0.1 to 200 parts, preferably 1 to 200 parts of the outer wall powder, per 100 parts of the core powder. At a level of less than 0.1 part, the effect of the sheath powder cannot be exhibited. When a powder surface modifier is used, it is used in an amount of 0.01% to 10% by weight, based upon the core powder and the sheath powder.

In the practice of the present invention, the use of the composite powder having a modified surface is preferable from the viewpoint of the effects thereof. As a method of preparing the surface modified composite powder, the composite powder is prepared after the core powder and/or the sheath powder are surface modified; the composite powder is prepared by adding a surface modifier to the core powder and the sheath powder during the composite powder preparation step; and the surface modification is effected after the preparation of the composite powder and the like.

Typical examples of the surface modifier include oils such as ester oils, hydrocarbon oils, fatty acids, and silicone oils (e.g., methylhydrogen polysiloxane, 1,3,5,7-tetramethylcyclotetrasiloxane, dimethylpolysiloxane, methylphenylpolysiloxane); waxes such as whale wax, Japan wax, shellac, beeswax, lanolin, carnauba wax, and canderilla; coupling agents such as silane coupling agents (e.g., vinyl trichrolosilane, triethoxy vinylsilane, 3-chloropropyl trimethoxysilane); sillylating agents (e.g., trimethylchloro silane, hexamethyl disilazane, diethylamino trimethyl silane), titanate coupling agents (e.g., methyltrichloro titanium,

3

isopropyltriisostearoyl titanate, tetraoctyl bis (ditridecyl phosphite) titanate, bis(dioctyl pyrophosphate)-ethylene titanate); monomers forming synthetic polymers (e.g. polyamine forming monomers such as caprolactum), polyolefin forming monomers such as ethylene and propylene, styrene monomers, polyester forming monomers such as divalent aromatic phenols and bis phenol A, acryl resin forming monomers such as methyl methacrylate, methacrylic acid, butyl methacrylate, epoxy resin monomers such as bisphenol A and epichlorohydrin, fluorine resin monomers, silicone resin monomers; surfactants such as alkylallyl sulfonates, sulfuric acid ester salts of higher alcohols, phosphoric acid ester salts of higher alcohols; silicone surfactants such as polyether modified silicone, amino modified silicone, alkyl modified silicone, alcohol modified silicone, and carboxylic acid modified silicone; fluorine surfactants such as perfluoroalkyl carboxylic acid salts, perfluoroalkyl phosphoric acid esters, perfluoroalkyl carboxylic acid salts, perfluoroalkyl trimethylammonium salts, perfluoroalkyl betains, perfluoroalkylamine oxides, and perfluoroalkyl ethylene oxide addition products; metal soaps such as aluminum stearate, calcium stearate, magnesium stearate, zinc stearate, aluminum myristate, calcium myristate, magnesium myristate, zinc myristate, aluminum palmitate, calcium palmitate, magnesium palmitate, zinc palmitate, zinc laurate, zinc undecylate, and calcium acetate; silicone resins; silicone rubber; gelatin; collagen; keratin; and fibroin.

As the surface modification method, any connectional modification methods may be used in the present invention. Examples of such methods are modification by coating, topochemical modification, mechanochemical modification, modification by encapsulation, radiation modification, and plasma irradiation modification. These methods are carried out under, for example, the vapor phase, the liquid phase or in vacuo. Furthermore, the composite powder may be modified only by, for example, plasma irradiation, without using the surface modifier.

The amount of the composite powder formulated in the present invention is preferably 0.1% to 30% by weight in the total amount of the aerosol composition, more preferably 0.3% to 20% by weight: If the amount is less than 0.1%, the effect required from the powder formulation cannot be obtained, and the effect will be unchanged if it is formulated in excess of 30% by weight.

The propellant usable in the present invention is one or more of propane, isobutane, n-butane, and LPG, which is a mixture thereof, chlorinated hydrocarbons such as methyl chloride and methylene chloride and dimethyl ether, and these propellants can be further used in combination with chlorofluoro carbon gases such Freon 11*, Freon 12*, Freon 21*, Freon 113*, and Freon 114*.

When chlorofluoro carbon gas used in combination, if the average specific gravity is 1.2 or less, the required dispersibility effect is obtained. Accordingly, when the specific gravity of the propellant becomes 1.2 or less, if the powder formulated in the powder spray of the prior art is now formulated, although the dispersibility is worsened it can be improved by a formulation of the composite powder of the present invention. Also, to control the inner pressure and dissolve the formulated product, a solvent as represented by an alcohol also can be formulated in the composition. Examples of such solvents are alcohol, n-pentane, isopentane, n-hexane, cyclohexane, isohexane, n-heptane, isooctane, 2,2-dimethylbutane, petroleum ether, petroleum benzene, ligroin, 2-pentene, methyl ethyl ether, and diethyl ether. Typically, inner pressure is 0.1 to 2.5 $kg/cm^2$ G (gauge) at a temperature of 20 °C.

The amount of propellant formulated is preferably 5 to 99% by weight of the total amount of the aerosol composition, more preferably 15 to 95% by weight. A good jetting state cannot be obtained at a level of less than 5% by weight.

In the composition of the present invention, in addition to the above components, any of the optional components known in the art can be formulated.

Examples of such formulation components include water; oils and fats such as avocado oil, almond oil, olive oil, grapeseed oil, sesame oil, Sasanqua oil, safflower oil, soybean oil, Tsubaki (camellia) oil, corn oil, rapeseed oil, persic oil, castor oil, sunflower oil, cottonseed oil, peanut oil, cacao oil, palm oil, coconut oil, tallow, fish fat, hardened oil, turtle oil, lard, mink oil, and egg yolk oil; waxes such as whale wax, shellac, beeswax, lanolin, liquid lanolin, carnauba wax, and canderilla wax; hydrocarbons such as liquid paraffin, liquid polyisobutylene, squalane, pristane, petrolatum, paraffin, and ceresin; silicone oils such as dimethyl polysiloxane, methyl phenyl polysiloxane, methylhydrogen polysiloxane, octamethyl cyclo tetrasiloxane, and decamethyl cyclopentasiloxane; fatty acids such as succinic acid, tartaric acid, citric acid, undecyleic acid, lauric acid, myristic acid, palmitic acid, stearic acid, isostearic acid, oleic acid, linoleic acid, ricinoleic acid, and behenic acid; alcohols such as ethanol, isopropanol, lauryl alcohol, cetanol, 2-hexyldecaol, stearyl alcohol, isostearyl alcohol, oleyl alcohol, and lanolin alcohol; polyhydric alcohols such as ethylene glycol, diethylene glycol monoethyl ether, triethylene glycol, polyethylene glycol, propylene glycol, 1,3-butylene glycol, glycerine, and batyl alcohol; sugars such as glucose, sucrose, lactose, xylitol, sorbitol, mannitol, and

*Trade Mark

4

maltitol; esters such as diisopropyl adipate, hexyldecyl isostearate, cetyl isooctanoate, olecyl oleate, decyl oleate, lanolin acetate, butyl stearate, isopropyl myristate, diethyl phthalate, and hexyl laurate; metal soaps such as aluminum stearate, magnesium stearate, zinc stearate, aluminum myristate, calcium myristate, magnesium myristate, zinc myristate, aluminum palmitate, calcium palmitate, magnesium palmitate, zinc palmitate, zinc laurate, zinc undecylate, and calcium acetate; natural water soluble polymeric compounds such as gum arabic, sodium alginate, casein, carageehnan, karaya gum, agar, quinceseed, gelatin, dextrin, starch, tragacanth, and pectin; natural dyes such as chlorophil and $\beta$-carotin; sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; glycerol polyglycerol fatty acids such as mono cottonseed oil fatty acid glycerol, monoerucic acid glycerol, sesquioleic acid glycerol, monostearic acid glycerol, $\alpha,\alpha'$-oleic acid pyroglutamic acid glycerol, monostearic acid glycerol malic acid; propylene glycol fatty acid esters such as propylene glycerol monostearate; lipophilic nonionic surfactants such as hydrogenated castor oil derivatives, glycerol alkylethers; polyoxyethylene (i.e., POE) fatty acid esters such as POE sorbitan monooleate, POE sorbitan monostearate, POE sorbitan monooleate, POE sorbitan pentaoleate, and POE sorbitan tetraoleate; POE sorbitol fatty acid esters such as POE sorbitol monolaurate, POE sorbitol monooleate, POE sorbitol pentaoleate, POE sorbitol monostearate; POE glycerol fatty acid esters such as POE glycerol monostearate, POE glycerol monoisostearate, and POE glycerol triisostearate; POE fatty acid esters such as POE monooleate, POE distearate, POE monodioleate and ethylene glycol distearate; POE alkylethers such as POE lauryl ether, POE oleyl ether, POE stearyl ether, POE behenyl ether, POE 2-octyldodecyl ether, POE chlestanol ether; POE alkyl phenyl ethers such as POE octyl phenyl ether, POE nonyl phenyl ether, and POE dinonyl phenyl ether; Pluronic type surfactants such as Pluronic; POE•POP alkyl ethers such as POE•POP cetyl ether, POE•POP 2-decyl tetradecyl ether, POE•POP monobutyl ether, POE•POP hydrogenated lanolin, and POE•POP glycerol ether; tetra POE•tera POP ethylenediamine condensates such as Tetronic; POE castor oil hydrogenated castor oil derivatives such as POE castor oil, POE hydrogenated castor oil, POE hydrogenated castor oil monoisostearate, POE hydrogenated castor oil - isostearate, POE hydrogenated castor oil monopyroglutamic acid monoisostearic acid diester, and POE hydrogenated castor oil maleic acid; POE beeswax•lanolin derivatives such as POE sorbitol beeswax; alkanol amides such as coconut oil fatty acid diethanolamide, lauric acid monoethanol amide; hydrophilic nonionic surfactants such as POE propylene glycol fatty acid esters, POE alkylamines, POE fatty acid amides, sucrose fatty acid esters, POE nonylphenyl formaldehyde condensates, alkylethoxydimethyl amine oxides, and trioleyl phosphoric acid; fatty acid soaps such as soap bases, sodium laurate and sodium palmitate; higher alkyl sulfuric acid ester salts such as sodium lauryl sulfate, potassium lauryl sulfate; alkyl ether sulfuric acid ester salts such as POE lauryl sulfate triethanolamine and sodium POE lauryl sulfate; N-acyl sarcosinic acids such as sodium lauroyl sarcosinate; higher fatty acid amide sulfonic acid salts such as sodium N-myristoyl-N-methyl taurine, sodium coconut oil fatty acid methyltauride, and sodium lauryl methyltauride; phosphoric acid ester salts such as sodium POE oleyl ether phosphate and sodium POE stearyl ether phosphate; sulfosuccinic acid salts such as sodium di-2-ethylhexyl sulfosuccinate, sodium monolauroyl monoethanolamide polyoxyethylene sulfosuccinate, and sodium lauryl polypropyleneglycol sulfosuccinate; alkylbenzene sulfonic acid esters of, for example, linear dodecylbenzene sulfonic acid such as sodium linear dodecylbenzene sulfonate and triethanolamine linear dodecylbenzene sulfonate; N-acylglutamic acid salts such as monosodium N-lauroyl glutamic acid, disodium N-stearoyl glutamic acid, and monosodium N-myristoyl-L-glutamate; higher fatty acid ester sulfuric acid ester salts such as sodium hydrogenated coconut oil fatty acid glycerol sulfate; sulfated oil such as Turkey red oil; anionic surfactants such as POE alkyl ether carboxylic acids, POE alkyl allyl ether carboxylic acid salts, $\alpha$-olefin sulfonic acid salts, higher fatty acid ester sulfonic acid salts, secondary alcohol sulfuric ester salts, higher fatty acid alkylolamide sulfuric acid ester salts, sodium lauroyl monoethanolamide succinate, N-palmytoyl aspartic acid ditriethanolamine, and sodium casein; silicone surfactants such as polyether modified silicone, amino modified silicone, alkyl modified silicone, alcohol modified silicone, and carboxylic acid modified silicone; fluorine containing surfactants such as perfluoroalkyl carboxylic acid salts, perfluoroalkyl phosphoric acid esters, perfluoroalkyl carboxylic acid salts, perfluoroalkyl trimethyl ammonium salts, perfluoroalkyl betains, perfluoroalkylamine oxide, and perfluoroalkyl EO addition products; preservatives such as butyl p-oxybenzoate, propyl p-oxybenzoate, and methyl p-oxybenzoate;

vitamins such as vitamin A, vitamin D, vitamin E, and vitamin K; hormones such as estradiol, ethynylestradiol, and cortisone;

antisweat agents such as aluminum hydroxychloride, aluminum chloride, aluminum sulfate, basic aluminum bromide, aluminum phenolsulfonic acid, tannic acid, aluminum naphthalenesulfonic acid, and basic aluminum iodide; UV-absorbers such as urocanic acid and cynoxate; antiphlogistics such as allantoin, aloe powder, and guaizulene; sterilizers such as 3,4,4-trichlorocarbanilide (T.C.C.), triethylcitrate (T.E.C.), benzalkonium chloride, benzotonium chloride, alkyltrimethylammonium chloride, resorcin, phenol, sorbic acid, salicylic acid, and hexachlorophen; essential oils which are natural plant perfumes such as lavender, lemon, jasmin, mint, peppermint, rose, and camphor; animal perfumes such as musk, civet, and castoreum, and other synthetic perfumes; and a compressed gas such as carbon dioxide gas, nitrogen gas, and nitrous oxide gas.

The present invention provides an aerosol composition having an excellent powder dispersibility and useability by reducing the amount of chlorofluoro carbon gas or without using chlorofluoro carbon gas, and thus meets current requirements to restrict the use of this gas due to its influence on the ozone layer.

Examples

The present invention will now be further illustrated by, but is by no means limited to, the following Examples, wherein all parts and percentages are expressed on a weight basis unless otherwise noted.

Evaluation test methods used hereinbelow will be described.

Powder dispersibility test

Powder and a propellant were filled in an aerosol glass bottle, and after shaking, the time in which the powder sedimented at the bottom of the bottle was measured.

Useability test

Each sample was sprayed on the forearm for 3 seconds, and the extendability of the powder was evaluated. The evaluation standards are as follows.

⊙    ... very well extended and free flowing
o    ... slightly extended and free flowing
△    ... not well extended
x    ... very poorly extended

6

Examples 1 - 2 and Comparative Examples 1 - 4

Table 1 shows Examples and Comparative Examples.

Table 1

| Starting material | Example 1 | Comparative Example 1 | Comparative Example 2 | Example 2 | Comparative Example 3 | Comparative Example 4 |
|---|---|---|---|---|---|---|
| Composite powder [spherical nylon 12 (5 μm) : silica (0.1 μm) = 70:30] | 2 | - | - | 2 | - | - |
| Mixed powder [spherical nylon 12 (5 μm) : silica (0.1 μm) = 70:30] | - | 2 | - | - | 2 | - |
| Silica (0.1 μm) | - | - | 0.6 | - | - | 0.6 |
| LPG (isobutane) | 98 | 98 | 99.4 | - | - | - |
| LPG (isobutane)/Freon 11, 12 * (50/50) = 50/50 | - | - | - | 98 | 98 | 99.4 |
| Time until sedimentation occurred after shaking (sec.) | 30 sec. | 15 sec. | 7 sec. | 34 sec. | 18 sec.* | 10 sec. |
| Useability | ○ | △ | × | ○ | △ | × |

*only silica sedimented

specific gravity of propellant liquid (20°C)

LPG (isobutane)  0.56

LPG/*Freon(50/50)  0.98

* Trade Mark

As shown in Table 1, in Examples 1 and 2, the time taken for the powder to precipitate after shaking was remarkably lengthened. In the case of the propellant liquid with a specific gravity of 1.2 or less, those formulated with the composite powder had an extremely good dispersibility, with the sedimentation time being prolonged 3-fold to 4-fold compared to the silica formulated product of the Comparative Examples. Also, the useability of the present Examples was found to be significantly better, compared with Comparative Examples formulated with conventional silica.

7

Example 3: Deodorant Spray

| (1) | Composite powder [spherical methyl-polysilocane (9 $\mu$m) : magnesium metalsilicate aluminate (0.4 $\mu$m) : polymethyl methacrylate (0.7 $\mu$m) = 75:20:5] | 5.0 |
|---|---|---|
| (2) | Isopropyl myristate | 0.6 |
| (3) | Alcohol | 10.0 |
| (4) | Aluminum hydroxychloride | 0.2 |
| (5) | Perfume | 0.2 |
| (6) | L.P.G. (isobutane) | 50.0 |
| (7) | *Freon 12 | 14.0 |
| (8) | *Freon 114 | 20.0 |
| Average specific gravity of propellant | | 0.905 |

* Trade Mark

After the components (1) to (5) were filled in an aerosol can, a valve was mounted and crimped, and the components (6) to (8) were filled under pressure to obtain a deodorant spray. The spray had a good powder dispersibility and useability.

Example 4: Cologne Powder Spray

| (1) | Composite powder [plate polyethylene (8 $\mu$m): zinc white (0.05 $\mu$m) = 60:40] | 15.0 |
|---|---|---|
| (2) | Perfume | 3.0 |
| (3) | Polymethylsiloxane | 1.0 |
| (4) | L.P.G. [propane (10%)/n-butane (90%)] | 51.0 |
| (5) | Dimethyl ether | 30.0 |
| Average specific gravity of propellant | | 0.605 |

After (1) to (3) were filled in an aerosol can, a valve was mounted and crimped, and the components (4) and (5) were filled under pressure to obtain a cologne powder spray. The spray was found to have a good powder dispersibility and useability.

Example 5: Deodorant Spray

| (1) | Composite powder [spherical nylon 6 (7 $\mu$m) : silica (0.2 $\mu$m) = 70:30] | 10.0 |
|---|---|---|
| (2) | Benzotonium chloride | 0.05 |
| (3) | Alcohol | 20.0 |
| (4) | Basic aluminum bromide | 0.5 |
| (5) | Deionized water | 5.0 |
| (6) | Dimethyl ether | 34.45 |
| (7) | L.P.G. (isobutane) | 30.0 |
| Average specific gravity of propellant | | 0.613 |

After the components (1) to (5) were filled in an aerosol can, a valve was mounted and crimped, and the components (6) and (7) were filled under pressure to obtain a deodorant spray. The spray was found to have a good powder dispersibility and useability.

Example 6: Powder Cologne Spray

| (1) | Composite powder [particulate cellulose (20 $\mu$m) : talc (2 $\mu$m) = 74:26] | 3.0 |
|-----|---|---|
| (2) | Dipropylene glycol | 1.0 |
| (3) | Ethyl alcohol | 53.6 |
| (4) | Water | 12.0 |
| (5) | Camphor | 0.2 |
| (6) | Perfume | 0.2 |
| (7) | Dimethyl ether | 30.0 |
| Average specific gravity of propellant | | 0.66 |

After the components (1) to (6) were homogeneously mixed, the mixture was filled in an aerosol can and a valve was mounted and crimped. After crimping, (7) was filled under pressure to obtain a powder cologne spray. The spray was found to have a good powder dispersibility and useability.

Example 7: Deodorant Powder Spray

| (1) | Composite powder [spherical polystyrene (50 $\mu$m) : hydroxyapatite (0.3 $\mu$m) = 81:19 | 7.0 |
|-----|---|---|
| (2) | Ethyl alcohol | 39.8 |
| (3) | Water | 10.0 |
| (4) | Propylene glycol | 3.0 |
| (5) | Aluminum hydroxychloride (50%) | 10.0 |
| (6) | Perfume | 0.2 |
| (7) | Dimethyl ether | 25.0 |
| (8) | L.P.G. (butane) | 5.0 |
| Average specific gravity of propellant | | 0.65 |

The components (1) to (6) were homogeneously mixed and then filled in a aerosol can, and a valve was mounted and crimped, followed by filling (7) and (8) to obtain a deodorant powder spray. The spray was found to have a good powder dispersibility and useability.

Example 8: Powder Spray

| (1) | Composite powder (granular tetrafluoroethylene (1 $\mu$m) : kaolin (0.1 $\mu$m) = 91:9 | 4.7 |
|-----|---|---|
| (2) | Ethyl alcohol | 27.0 |
| (3) | Water | 9.0 |
| (4) | Potassium glycyrrhizinate | 0.1 |
| (5) | Perfume | 0.2 |
| (6) | Freon 11/12 (80/20) | 36.0 |
| (7) | Dimethyl ether | 18.0 |
| Average specific gravity of propellant | | 1.19 |

After the components (1) to (5) were mixed, the mixture was filled in an aerosol can, and a valve was mounted and crimped, followed by filling (6) and (7) under pressure to obtain a powder spray. The spray was found to have a good powder dispersibility and useability.

EP 0 348 091 B1

Example 9: Powder Spray for Systemic Use

| | | |
|---|---|---|
| (1) | Composite powder [granular chitosan (30 $\mu$m) : magnesium silicate aluminate (0.5 $\mu$m) = 69:31] | 10.0 |
| (2) | Mentol | 0.1 |
| (3) | Camphor | 0.2 |
| (4) | Eucalyptus oil | 0.1 |
| (5) | Ethanol | 39.6 |
| (6) | Freon 12 | 10.0 |
| (7) | Dimethyl ether | 40.0 |
| | Average specific gravity of propellant | 0.794 |

A solution of (2) to (4) dissolved in the components (1) and (5) was filled in an aerosol can and a valve was mounted and crimped, followed by filling (6) and (7) under pressure to obtain a powder spray for systemic use. The spray was found to have a good powder dispersibility and useability.

Example 10: Deodorant Cologne Spray

| | | |
|---|---|---|
| (1) | Composite powder [particulate fibroin (25 $\mu$m) : particulate chitin (2 $\mu$m) : light calcium carbonate (1 $\mu$m) = 69:19:12 | 5.0 |
| (2) | Trichlosane | 0.1 |
| (3) | Ethanol | 30.0 |
| (4) | Aluminum hydroxychloride (50%) | 10.0 |
| (5) | Water | 10.0 |
| (6) | Perfume | 0.1 |
| (7) | Polyoxyethylene (60 mole adduct) hardened castor oil derivative | 0.5 |
| (7) | Dimethyl ether | 40.0 |
| (8) | L.P.G. (butane) | 4.3 |
| | Average specific gravity of propellant | 0.652 |

After (2), (6) and (7) were dissolved in the components (1) and (3), (4) and (5) were added, and the mixture filled in an aerosol can. A valve was mounted and crimped, followed by filling (8) and (9) under pressure to obtain a deodorant cologne spray. The spray was found to have a good powder dispersibility and useability.

Example 11: Deodorant Powder Spray

| | | |
|---|---|---|
| (1) | Composite powder [spherical polymethyl methacrylate (0.5 $\mu$m) : aluminum hydroxychloride (0.05 $\mu$m)] = 60:40 | 15.0 |
| (2) | Talc | 1.0 |
| (3) | Isopropyl myristate | 0.5 |
| (4) | Tetra-2-ethylhexanoic acid diglycerolsorbitane | 0.5 |
| (5) | Dimethyl ether | 30.3 |
| (6) | L.P.G. | 50.0 |
| | Average specific gravity of propellant | 0.677 |

After the components (1) to (4) were homogeneously mixed, the mixture was filled in an aerosol can. A valve was mounted and crimped, followed by filling (5) and (6) under pressure to obtain a deodorant powder spray. The spray was found to have a good powder dispersibility and useability.

10

Example 12: Deodorant Spray

| (1) | Composite powder [spherical crosslinked polymethyl methacrylate (15 $\mu$m) : aluminum hydroxy chloride (0.5 $\mu$m) : silicone resin = 70:26:4] | 7.0 |
|---|---|---|
| (2) | Isopropyl myristate | 0.7 |
| (3) | Ethanol | 8.0 |
| (4) | Perfume | 0.1 |
| (5) | L.P.G. [n-butane (75%)/isobutane (25%)] | 84.2 |
| | Average specific gravity of propellant | 0.588 |

After the components (1) to (4) were uniformly mixed and filled in an aerosol can, a valve was mounted and crimped, and the component (5) was filled under pressure to obtain a deodorant spray.

Example 13: Cologne Powder Spray

```
(1)   Composite powder [granular nylon 46
      (7 µm) : basic aluminum (0.2 µm) :
      squalane : carnauba wax


      = 80:25:2.5:2.5]                          13.0
(2)   Perfume                                    3.0
(3)   Polymethylphenylsiloxane                   2.0
(4)   L.P.G. [n-butane (90%)/propane (10%)]     50.0
(5)   Dimethyl ether                            30.0
      Average specific gravity of propellant   0.572
```

After (1) to (3) were filled in an aerosol can, a valve was mounted and crimped, and the components (4) and (5) were filled under pressure to obtain a cologne powder spray.

Example 14: Foot Spray

| (1) | Composite powder [granular crosslinked polystyrene (12 $\mu$m) : aluminum citrate (0.4 $\mu$m) : calcium stearate (0.7 $\mu$m) = 64:35.2:0.8] | 10.0 |
|---|---|---|
| (2) | Composite powder [spherical benzoguanamine resin (10 $\mu$m) : zinc oxide (0.04 $\mu$m) -75:25] | 10.0 |
| (3) | Dimethyl polysiloxane | 5.0 |
| (4) | Perfume | 0.9 |
| (5) | L.P.G. [Isobutane (80%)/propane (20%)] | 74.1 |
| | Average specific gravity of propellant | 0.548 |

After (1) to (4) were uniformly mixed and filled in an aerosol can, a valve was mounted and crimped, and the component (5) was filled under pressure to obtain a foot spray.

The powder dispersibility and useability of the products obtained in Examples 12, 13, and 14 were evaluated as mentioned above. The results are shown in Table 2.

Table 2

|  | Example | | |
|---|---|---|---|
|  | 12 | 13 | 14 |
| Time until sedimentation occurred after shaking (sec) | 123 | 84 | 98 |
| Useability | ◎ | ◎ | ◎ |

As is clear from the results shown in Table 2, the products of Examples 12 to 14 had an excellent dispersibility and useability.

Example 15: Deodorant Spray

| (1) | Composite powder [flakypolyethylene coated with high viscosity silicone (6 $\mu$m) : hexamethyldisilazane treated zinc oxide (0.2 $\mu$m) = 79:21 | 11.0 |
|---|---|---|
| (2) | Ethanol | 38.0 |
| (3) | Water | 9.0 |
| (4) | Propylene glycol | 3.0 |
| (5) | Perfume | 0.3 |
| (6) | L.P.G. [n-butane (52.5%)/isobutane (17.5%)/propane (30.0%)] | 38.7 |
|  | Average specific gravity of propellant | 0.553 |

After the components (1) to (5) were uniformly mixed and filled in an aerosol can, a valve was mounted and crimped, and the component (6) was filled under pressure to obtain a deodorant spray. The spray obtained was found to have a good powder dispersibility, useability, and deodorizing property.

Example 16: Powder Spray

```
(1)   Composite powder [spherical cured
      bisphenol A-epichlohydrin copolymer
      powder (18 µm) : mica coated with



      high viscosity methylphenyl poly-
      siloxane (0.9 µm) = 61:39]              14.0
(2)   Talc                                     1.2
(3)   Isopropyl myristate                      0.8
(4)   L.P.G. [n-butane (45%)/isobutane
      (15%)/propane (40%)                      84.0
      Average specific gravity of propellant   0.545
```

After the components (1) to (3) were uniformly mixed and filled in an aerosol can, a valve was mounted and crimped. After crimping, the component (4) was filled under pressure to obtain a powder spray. The spray was found to have a good powder dispersibility and useability.

12

Example 17: Powder Spray for Systemic Use

| | | |
|---|---|---|
| (1) | Composite powder [granular polypropylene (18 $\mu$m) : zinc oxide (0.05 $\mu$m) : silicone resin = 71:28:1] | 11.0 |
| (2) | Mentol | 0.2 |
| (3) | Camphor | 0.1 |
| (4) | Eucalyptus oil | 0.1 |
| (5) | Ethanol | 38.6 |
| (6) | L.P.G. [n-butane (49%)/isobutane (16%)/propane (35%)] | 37.5 |
| (7) | Freon 11 | 12.5 |
| | Average specific gravity of propellant | 0.776 |

After the components (1) to (5) were mixed, the mixture was filled in an aerosol can, and a valve was mounted and crimped, followed by filling (6) and (7) under pressure to obtain a powder spray. The spray was found to have a good powder dispersibility and useability.

Example 18: Powder Spray

| | | |
|---|---|---|
| (1) | Composite powder [spherical chitosan (20 $\mu$m) : magnesium oxide (0.6 $\mu$m) : zinc myristate (0.8 $\mu$m) = 79:20.5:0.5] | 13.5 |
| (2) | Spherical silica | 0.5 |
| (3) | Squalane | 2.5 |
| (4) | Methylphenyl polysiloxane | 2.0 |
| (5) | Isopentane | 16.3 |
| (6) | L.P.G. [n-butane (75%)/isobutane (25%)] | 65.2 |
| Vapor pressure of a mixture of (5) and (6): 1.1 kg/cm$^2$G (gauge) at 20°C | | |

The components (1) and (2) and the components (3) and (4) were separately uniformly mixed. The resultant mixtures and the component (5) were then filled in an aerosol can, and a valve was mounted and crimped, followed by filling the component (6) under pressure to obtain a powder spray.

Example 19: Foot Spray

| | | |
|---|---|---|
| (1) | Composite powder [granular polyvinylidene chloride (7 $\mu$m) : calcium oxide treated with perfluoroalkylphosphoric acid ester (0.9 $\mu$m) = 61:39] | 13.0 |
| (2) | Aluminum stearate | 0.4 |
| (3) | Aluminum hydroxy chloride | 1.0 |
| (4) | Octamethyl cyclotetrasiloxane | 2.5 |
| (5) | Decamethyl cyclopentasiloxane | 2.5 |
| (6) | n-pentane | 48.36 |
| (7) | LPG [propane (43%)/isobutane (16%)/n-butane (41%)] | 32.24 |
| Vapour pressure of a mixture of (6) and (7): 1.4 kg/cm$^2$G (20°C) | | |

The components (1) to (3) and the components (4) and (5) were separately uniformly mixed and then filled, together with the component (6), in an aerosol can, and a valve was mounted and crimped, followed by filling the component (7) under pressure to obtain a foot spray.

## Claims

**1.** An aerosol composition comprising a composite powder and at least one propellant selected from the group consisting of propane, n-butane, isobutane, a liquefied petroleum gas, a chlorinated hydrocarbon, and dimethyl ether, said composite powder comprising a core powder having an average particle size of 0.1 to 100 $\mu$m consisting of a polymer and a sheath powder having an average particle size of 1/5 or less of that of the core powder attached to the surface of the core powder.

**2.** An aerosol composition as claimed in claim 1, further comprising a chlorofluoro carbon gas.

**3.** An aerosol composition comprising at least one propellant selected from the group consisting of propane, n-butane, isobutane, and a liquefied petroleum gas, at least one solvent selected from the group consisting of n-pentane, isopentane, and n-hexane, and a composite powder comprising a core powder having an average particle size of 0.1 to 100 $\mu$m consisting of a polymer and a sheath powder having an average particle size of 1/5 or less of that of the core powder attached to the surface of the core powder.

**4.** An aerosol composition as claimed in claim 3, wherein the vapor pressure of a mixture of the propellant and the solvent is 0.1 to 2.5 kg/cm$^2$G at 20°C.

**5.** An aerosol composition as claimed in claims 3 or 4, further comprising a chlorofluoro carbon gas.

**6.** An aerosol composition as claimed in any one of the preceding claims, wherein said core powder consists of at least one polymer selected from the group consisting of polyamides, polyolefins, polystyrene, polyesters, acrylic resins, epoxy resins, fluorine type resins, silicone type resins, phenolic resins, vinyl resins, polyurethane, cellulose, chitin, chitosan, fibroin, keratin, and natural rubbers.

## Patentansprüche

**1.** Aerosol-Zusammensetzung mit einem Verbundpulver und mindestens einem Treibmittel, ausgewählt aus der Gruppe bestehend aus Propan, n-Butan, Isobutan, einem verflüssigten Erdölgas, einem chlorierten Kohlenwasserstoff sowie Dimethylether, wobei das Verbundpulver aufweist ein Kernpulver mit einer durchschnittlichen Teilchengröße von 0,1 bis 100 $\mu$m, bestehend aus einem Polymeren und ein Hüllenpulver mit einer durchschnittlichen Teilchengröße von 1/5 oder Weniger der Teilchengröße des Kernpulvers, das an die Oberfläche des Kernpulvers angefügt ist.

**2.** Aerosol-Zusammensetzung nach Anspruch 1, die weiterhin ein Chlorofluorokohlenstoffgas enthält.

**3.** Aerosol-Zusammensetzung mit mindestens einem Treibmittel, ausgewählt aus der Gruppe bestehend aus Propan, n-Butan, Isobutan und einem flüssigen Erdölgas, mindestens einem Lösungsmittel, ausgewählt aus der Gruppe bestehend aus n-Pentan, Isopentan sowie n-Hexan sowie mit einem Verbundpulver aus einem Kernpulver mit einer durchschnittlichen Teilchengröße von 0,1 bis 100 $\mu$m, bestehend aus einem Polymer und einem Hüllenpulver mit einer durchschnittlichen Teilchengröße von 1/5 oder weniger der Teilchengröße des Kernpulvers, das an die Oberfläche des Kernpulvers angefügt ist.

**4.** Aerosol-Zusammensetzung nach Anspruch 3, bei dem der Dampfdruck einer Mischung aus dem Treibmittel und dem Lösungsmittel bei 0,1 bis 2,5 kg/cm$^2$G bei 20°C liegt.

**5.** Aerosol-Zusammensetzung nach Anspruch 3 oder 4, die weiterhin ein Chlorofluorokohlenstoffgas enthält.

**6.** Aerosol-Zusammensetzung nach einem der vorstehenden Ansprüche, in der das Kernpulver aus mindestens einem Polymeren besteht, das ausgewählt ist aus der Gruppe bestehend aus Polyamiden, Polyolefinen, Polystyrol, Polyestern, Acrylharzen, Epoxyharzen, Harzen vom Fluor-Typ, Harzen vom Silicon-Typ, phenolischen Harzen, Vinylharzen, Polyurethanen, Cellulose, Chitin, Chitosan, Fibroin, Keratin und natürlichen Gummis.

**Revendications**

1.  Une composition d'aérosol comprenant une poudre composite et au moins un propulseur choisi dans le groupe constitué par propane, n-butane, isobutane, un gaz de pétrole liquéfié, un hydrocarbure chloré et du diméthyléther, cette poudre composite comprenant une poudre formant un noyau dont la dimension moyenne des particules est comprise entre 0,1 et 100 $\mu$m, constituée d'un polymère, et d'une poudre d'enrobage revêtant la surface du noyau dont la dimension moyenne des particules est égale ou inférieure au 1/5 de celle de la poudre dont est formé le noyau.

2.  Une composition d'aérosol selon la revendication 1, caractérisée en ce qu'elle comprend en outre un chlorofluorocarbone gazeux.

3.  Une composition d'aérosol comprenant au moins un propulseur choisi dans le groupe constitué par propane, n-butane, isobutane et un gaz de pétrole liquéfié, au moins un solvant choisi dans le groupe constitué par n-pentane, isopentane et n-hexane et une poudre composite comprenant une poudre formant le noyau, dont la dimension moyenne des particules est comprise entre à,1 et 100 $\mu$m, constituée d'un polymère, et un enrobage de poudre revêtant la surface du noyau, dont la dimension moyenne des particules est égale ou inférieure au 1/5 de celle de la poudre formant le noyau.

4.  Une composition d'aérosol selon la revendication 3, caractérisée en ce que la tension de vapeur d'un mélange du propulseur et du solvant est comprise entre 0,1 et 2,5 kg/cm$^2$G à 20°C.

5.  Une composition d'aérosol selon l'une quelconque des revendications 3 et 4, caractérisée en ce qu'elle comprend en outre un chlorofluorocarbone gazeux.

6.  Une composition d'aérosol selon l'une quelconque des revendications précédentes, caractérisée en ce que la poudre formant le noyau est au moins constitué d'un polymère choisi dans le groupe constitué par polyamides, polyoléfines, polystyrène, polyesters, résines acryliques, résines époxy, résines de type fluoré, résines de type silicone, résines phénoliques, résines vinyliques, polyuréthane, cellulose, chitine, chitosane, fibroine, kératine et caoutchoucs naturels.